Europäisches Patentamt

European Patent Office

Office européen des brevets

(19)

(11) Publication number: **0 326 418**
**A1**

## (12) EUROPEAN PATENT APPLICATION

(21) Application number: **89300827.6**

(22) Date of filing: **27.01.89**

(51) Int. Cl.⁴: **C 07 K 7/10**
**A 61 K 37/02**

(30) Priority: **29.01.88 US 150301**

(43) Date of publication of application:
**02.08.89 Bulletin 89/31**

(84) Designated Contracting States: **ES GR**

(71) Applicant: **THE UPJOHN COMPANY**
**301 Henrietta Street**
**Kalamazoo, Michigan 49001 (US)**

(72) Inventor: **Friedman, Alan R.**
**7646 Wendy Lane**
**Portage Michigan 49081 (US)**

(74) Representative: **Perry, Robert Edward et al**
**GILL JENNINGS & EVERY 53-64 Chancery Lane**
**London WC2A 1HN (GB)**

(54) **GRF analogs.**

(57) GRF PEPTIDES wherein a cysteic acid residue (Cya) is substituted for the amino acid in position R3 and/or R25.

EP 0 326 418 A1

Description

## GRF ANALOGS

The present invention relates to a peptide having influence on the function of the pituitary gland in humans and other animals, particularly mammals. In particular, the present invention is directed to peptides which promote the release of growth hormone by the pituitary gland.

### BACKGROUND OF THE INVENTION

Physiologists have long recognized that the hypothalamus controls the secretory functions of the adenohypophysis with the hypothalamus producing special substances which stimulate or inhibit the secretion of each pituitary hormone. In 1982, human pancreatic (tumor) releasing factors (hpGRF) were isolated from extracts of human pancreatic tumors, purified, characterized, synthesized, tested, and found to promote release of growth hormone (GH) by the pituitary. Since then, corresponding hypothalamic GH releasing factors from other species including the rat species, the porcine species, the ovine species, the bovine and caprine species and from the human species have also been characterized and synthesized. Human hypothalamic GRF(hGRF) has been found to have the same formula as hpGRF, namely: H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-NH$_2$.

Synthetic polypeptides have also been synthesized which are disclosed as growth hormone releasing factors (GRF).

Although there are a number of cysteic acid containing peptides described in the literature, only two have been reported in which cysteic acid replaced aspartic acid. Konig et al, Peptides 7: Suppl. 1, 61-67 (1986) reported that cysteic acid replacement of aspartic acid in secretin resulted in a peptide with 20-40% of the biological activity of the parent peptide. In the second case, Morley J.S., Proc. Roy. Soc. B 170, 97-111 (1968) reported that cysteic acid replacement of aspartic acid in a biologically active fragment of gastrin resulted in a peptide devoid of biological activity.

### SUMMARY OF THE INVENTION

The present invention particularly provides a natural or synthetic polypeptide which promotes the release of growth hormone by the pituitary gland (GRF PEPTIDE) and having at least one cysteic acid in place of the amino acid residue normally found at position 3 or 25 of the polypeptide

### DETAILED DESCRIPTION OF THE INVENTION

The term "GRF PEPTIDE", as used in the specification and claims, means a known polypeptide which is between about 27 and 44 residues in length and that promotes the release of growth hormone by the pituitary gland. Illustrative GRF PEPTIDES include the natural or synthetic polypeptides disclosed in US Patent Nos. 4,517,181, 4,518,586, 4,528,190, 4,529,595, 4,563,352, 4,585,756, 4,595,676, 4,605,643, 4,610,976, 4,626,523, 4,628,043, and 4,689,318; all of which are incorporated herein by reference. Felix, A., Wang, C.T., Heimer, E., Fournier, A., Bolin, D., Ahmed, M., Lambros, T., Mowles, T., and Miller, L., "Synthesis and Biological Activity of Novel Linear & Cyclic GRF Analogs", "Peptides, Proceedings of the 10th American Peptide Symposium", G. Marshall ed., pg. 465-467, Escom, Leiden, 1988; Tou, J.S., Kaempfe, L.A., Vineyard, B.D., Buonomo, F.C., Della-Fera, M.A., and Baile, C.A., "Amphiphilic Growth Hormone Releasing Factor Analogs. Peptide Design and Biological Activity in vivo", Biochem. Biophys. Res. Commun. 139 #2, pp. 763-770 (1986); Coy, D.H., Murphy, W.A., Sueires-Diaz, J., Coy, E.J., Lance, V.A., "Structure Activity Studies on the N-Terminal Region of Growth Hormone Releasing Factor", J. Med. Chem. 28, pp, 181-185 (1985); Felix, A.M., Heimer, E.P., Mowles, T.F., Eisenbeis, H., Leung, P., Lambros, T.J., Ahmed, M., and Wang, C.T., "Synthesis and Biological Activity of Novel Growth Hormone Releasing Factor Analogs", 19th European Peptide Symposium, Aug. 31-SEpt. 5, 1986; Velicelebi, G., Patthi, S., and Kaiser, E.T., "Design and Biological Activity of Analogs of Growth Hormone Releasing Factor with Potential Amphiphilic Helical Carboxyl Termini", Proc. Natl. Acad. Sci. U.S.A., 85, pp. 5397-5399 (1986); Ling., N., Baird, A., Wehrenberg, W.B., Munegumi, T., and Ueno, N., "Synthesis GRF Analogs as Competitive Antagonists of GRF Therapeutic Agents Produced by Genetic Engineering", Quo Vadis Symposium, Sanofi Group, May 29030, 1985, Toulouse-Labege, France, pp. 309-329. The term GRF PEPTIDE includes nontoxic salts thereof.

The nomenclature used to define the GRF PEPTIDE is that specified by Schroder & Lubke, "The Peptides", Academic Press (1965) wherein in accordance with conventional representation the amino group at the N-terminal appears to the left and the carboxyl group at the C-terminal to the right. Where the amino acid residue has isomeric forms, the L-form of the amino acid is being represented unless otherwise expressly indicated. The cysteic acid (sulfo alanine) residue is designated by the three letter code Cya.

The residue [beta(para-hydroxyphenyl)propionyl]- is identified herein as DesaminoTyr. The residue [alpha(para-hydroxyphenyl)acetyl]- is identified herein as Desamino para-hydroxyphenyl glycyl or Desamino PHPG.

The present invention provides synthetic GRF peptide analogs (GRF PEPTIDES) having the following formula:
H-R$_1$-R$_2$-R$_3$-Ala-R$_5$-R$_6$-R$_7$-R$_8$-R$_9$-R$_{10}$-R$_{11}$-R$_{12}$-R$_{13}$-R$_{14}$-R$_{15}$-Gln-R$_{17}$-R$_{18}$-R$_{19}$-R$_{20}$-R$_{21}$-R$_{22}$-R$_{23}$-R$_{24}$-R$_{25}$-R$_{26}$-R$_{27}$-R$_{28}$-R$_{29}$-Gln-Gln-Gly-Glu-Ser-Asn-Gln-Glu-Arg-Gly-Ala-R$_{40}$-Y wherein R$_1$ is Tyr, D-Tyr, His, D-His, Phe, D-Phe, (optionally substituted with either a C$^\alpha$Me or N$^\alpha$Me substituent),

DesaminoTyr, Desamino PHPG, or DesaminoHis; $R_2$ is Ala or D-Ala optionally substituted with either a $C^\alpha Me$ or $N^\alpha Me$ substituent, or Ser; $R_3$ is Asp, D-Asp, Glu or D-Glu; $R_5$ is Ile or Leu; $R_6$ is Phe or Tyr; $R_7$ is Ser or Thr; $R_8$ is Asn, D-Asn, Ser, D-Ser or Thr; $R_9$ is Ala or Ser; $R_{10}$ is Tyr, D-Tyr or Phe; $R_{11}$ is Arg or Lys; $R_{12}$ is Arg or Lys; $R_{13}$ is Ile, Leu or Val; $R_{14}$ is Leu, D-Leu, Ile or Val; $R_{15}$ is Ala, D-Ala, Gly, Gln or Leu; $R_{17}$ is Ala, Leu or D-Leu; $R_{18}$ is Ala, Leu, Ser or Tyr; $R_{19}$ is Ala or Ser; $R_{20}$ is Arg, or Lys; $R_{21}$ is Arg, or Lys; $R_{22}$ is Ala, Leu or Ile; $R_{23}$ is Leu, D-Leu, Ile or Val; $R_{24}$ is Gln or His; $R_{25}$ is Asp or Glu; $R_{26}$ is Ile, Leu or Val; $R_{27}$ is Ala, D-Ala, Nle, Val, Leu or Ile; $R_{28}$ is Asn, Ser or Ala; $R_{29}$ is Arg, D-Arg, Lys; $R_{40}$ is Arg, Arg-Ala, Arg-Ala-Arg or Arg-Ala-Arg-Leu; and Y signifies the carboxyl moiety of the amino acid residue at the C-terminal and is the radical $-COOR_a$, $-CR_aO, -CONHNHR_a$, $-CON(R_a) (R_b)$ or $-CH_2OR_a$, with $R_a$ and $R_b$ being lower alkyl or hydrogen; wherein cysteic acid residue (Cya) is substituted for Asp or Glu in position $R_3$ and/or $R_{25}$; or a biologically active fragment thereof extending from R at the N-terminus to a residue in any of positions 27 through 39 as its C-terminus; a non-toxic salt of the foregoing; or a Hse(lactone), HseOH or $HseN(R_a) (R_b)$ of the foregoing.

An embodiment of the invention is the peptide $Leu^{27}$ hGRF(1-44)$NH_2$ having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Leu-Asn-Arg-Gln-Gln-Gly-Glu-Arg-Asn-Gln-Glu-Gln-Gly-Ala-Lys-Val-Arg-Leu-$NH_2$;
wherein a cysteic acid residue (Cya) is substituted for Asp in position 3 and/or 25; or a biologically active fragment thereof extending from R at the N-terminus to a residue in any of positions 27 through 39 as its C-terminus; or a non-toxic salt of the foregoing.

A further embodiment of the invention is a 32 residue bGRF analog $Leu^{27}$ bGRF(1-32)$NH_2$ having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Leu-Asn-Arg-Gln-Gln-Gly-$NH_2$;
wherein Cya is substituted for Asp in position 3 and/or 25; or a non-toxic salt of the foregoing.

Another embodiment of the invention is the peptide $Val^{27}$ hpGRF(1-40)$NH_2$ having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Val-Ser-Arg-Gln-Gln-Gly-Glu-Ser-Asn-Gln-Glu-Arg-Gly-Ala-$NH_2$;
wherein Cya is substituted for Asp in position 3 and/or 25; or a biologically active fragment thereof extending from R at the N-terminus to a residue in any of positions 27 through 39 as its C-terminus; or a non-toxic salt of the foregoing.

A further embodiment of the invention is a 32 residue hpGRF analog $Leu^{27}$ hpGRF(1-32)$NH_2$ having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Leu-Ser-Arg-Gln-Gln-Gly-$NH_2$;
wherein Cya is substituted for Asp in position 3 and/or 25; or a non-toxic salt of the foregoing.

Further embodiments of the invention are: $DesaminoTyr^1$ $Arg^{12}$ $Arg^{21}$ $Leu^{27}$ hpGRF(1-44)OH having the formula:
DesaminoTyr-Ala-Asp-Ala-Ile-Phe-Thr-Asn-Ser-Tyr-Arg-Arg-Val-Leu-Gly-Gln-Leu-Ser-Ala-Arg-Arg-Leu-Leu-Gln-Asp-Ile-Leu-Ser-Arg-Gln-Gln-Gly-Glu-Ser-Asn-Gln-Glu-Arg-Gly-Ala-Arg-Ala-Arg-Leu-OH;
wherein Cya is substituted for Asp in position 3 and/or 25; or a non-toxic salt of the foregoing.
$D-Ala^2$, $Arg^{21}$, $Ile^{27}$, homo-$Ser^{44}$ rGRF(1-44)OH having the formula:
His-D-Ala-Asp-Ala-Ile-Phe-Thr-Ser-Ser-Tyr-Arg-Arg-Ile-Leu-Gly-Gln-Leu-Tyr-Ala-Arg-Arg-Leu-Leu-His-Glu-Ile-Ile-Asn-Arg-Gln-Gln-Gly-Glu-Arg-Asn-Gln-Glu-Gln-Arg-Ser-Arg-Phe-Asn-HomoSer-OH;
wherein Cya is substituted for Asp in position 3 and/or Glu in position 25; or a non-toxic salt of the foregoing.

A further embodiment of the invention is a 32 residue bGRF analog $DesaminoTyr^1$ $Ala^{15}$ $Leu^{27}$ bGRF(1-32)$NH_2$ having the formula:
DesaminoTyr-Ala-Asp-Ala-Ile-Phe-Thr-Asn-Ser-Tyr-Arg-Lys-Val-Leu-Ala-Gln-Leu-Ser-Ala-Arg-Lys-Leu-Leu-Gln-Asp-Ile-Leu-Asn-Arg-Gln-Gln-Gly-$NH_2$;
wherein Cya is substituted for Asp in position 3 and/or 25; or a non-toxic salt of the foregoing.

A preferred embodiment of the invention are the GRF peptides with Cya in position 3 and/or 25 and the following group(s) in the peptide:
$R_1$ is DesaminoTyr or Desamino PHPG;
$R_8$ and/or $R_{28}$ is Ser;
$R_{12}$ and $_{21}$ are Arg;
$R_{15}$ is Ala;
$R_{27}$ is Leu; and
$R_{33}$ is Gly-Hse(lactone), Gly-HseOH or Gly-HseN($R_a$) ($R_b$).

A preferred embodiment of the invention is a 33 residue bGRF analog $DesaminoTyr^1$ $Ser^8$ $Arg^{12}$ $Ala^{15}$ $Arg^{21}$ $Leu^{27}$ $Ser^{28}$ $Hse^{33}$ bGRF(1-33) lactone having the formula:
DesaminoTyr-Ala-Asp-Ala-Ile-Phe-Thr-Ser-Ser-Tyr-Arg-Arg-Val-Leu-Ala-Gln-Leu-Ser-Ala-Arg-Arg-Leu-Leu-Gln-Asp-Ile-Leu-Ser-Arg-Gln-Gln-Gly-Hse-lactone;
wherein Cya is substituted for Asp in position 3 and/or 25; or a non-toxic salt of the foregoing.

A preferred embodiment of the invention is a 33 residue bGRF analog $DesaminoTyr^1$ $Ser^8$ $Arg^{12}$ $Ala^{15}$ $Arg^{21}$ $Leu^{27}$ $Ser^{18}$ $Hse^{33}$ bGRF(1-33)$NH_2$ having the formula:
DesaminoTyr-Ala-Asp-Ala-Ile-Phe-Thr-Ser-Ser-Tyr-Arg-Arg-Val-Leu-Ala-Gln-Leu-Ser-Ala-Arg-Arg-Leu-Leu-Gln-Asp-Ile-Leu-Ser-Arg-Gln-Gln-Gly-Hse-$NH_2$;
wherein Cya is substituted for Asp in position 3 and/or 25; or a non-toxic salt of the foregoing.

A preferred embodiment of the invention is a 33 residue bGRF analog $Desamino-PHPG^1$ $Ser^8$ $Arg^{12}$ $Ala^{15}$ $Arg^{21}$ $Leu^{27}$ $Ser^{28}$ $Hse^{33}$ bGRF(1-33)OH the formula:
Desamino-$PHPG^1$-Ala-Asp-Ala-Ile-Phe-Thr-Ser-Ser-Tyr-Arg-Arg-Val-Leu-Ala-Gln-Leu-Ser-Ala-Arg-Arg-Leu-Leu-Gln-Asp-Ile-Leu-Ser-Arg-Gln-Gln-Gly-Hse-OH;
wherein Cya is substituted for Asp in position 3 and/or 25; or a non-toxic salt of the foregoing.

A further preferred embodiment of the invention is a 30 residue bGRF analog Desamino-PHPG[1] Ser[8] Arg[12] Ala[15] Arg[21] Leu[27] Ser[28] Hse[30] bGRF(1-30)NH(Ethyl) the formula: Desamino-PHPG[1]-Ala-Asp-Ala-Ile-Phe-Thr-Ser-Ser-Tyr-Arg-Arg-Val-Leu-Ala-Gln-Leu-Ser-Ala-Arg-Lys-Leu-Leu-Gln-Asp-Ile-Leu-Ser-Arg--Hse-NH(Ethyl); wherein Cya is substituted for Asp in position 3 and/or 25; or a non-toxic salt of the foregoing.

For purposes of production methodology the carboxy terminal residue is preferably homoserine, homoserine lactone, homoserine amide, or a lower $C_1-C_4$ alkyl, secondary or tertiary amides of homoserine.

The synthetic GRF peptide analogs are synthesized by a suitable method, including for example the methods disclosed in U.S. Patent 4,529,595 (Col 2, ln 35 to Col 5, ln 64) and US Patent 4,689,318 (Col 2, ln 23 to Col 9, ln 13), each of which are incorporated herein by reference.

Procedure A sets forth a preferred method for synthesizing GRF peptide analogs of the subject invention.

PROCEDURE A

The peptides are synthesized by solid-phase methodology utilizing an Applied Biosystems 430A peptide synthesizer (Applied Biosystems, Foster City, California) and synthesis cycles supplied by Applied Biosystems. Boc Amino acids and other reagents were supplied by Applied Biosystems and other commercial sources. Sequential Boc chemistry using double couple protocols are applied to the starting p-methyl benzhydryl amine resin for the production of C terminal carboxamides. For the production of C terminal acids, the corresponding PAM resin is used. Asparagine, Glutamine, Arginine, Histidine, Desamino para-hydroxyphenylglycine [alpha(para-hydroxyphenyl)acetic acid] and Desaminotyrosine [beta(parahydroxy phenyl propionic acid)] are coupled using preformed hydroxy benztriazole esters. All other amino acids are coupled using the preformed symmetrical Boc amino acid anhydrides.

The following side chain protection is used:
Arg, Tosyl
Asp, Benzyl
Cys, 4-Methyl Benzyl
Glu, Benzyl
His, Benzyloxymethyl
Lys, 2-Chloro Carbobenzoxy
Ser, Benzyl
Thr, Benzyl
Tyr, 4-Bromo Carbobenzoxy.

When symmetrical anhydride couplings are employed, the first coupling is carried out in dimethyl formamide (DMA) and the second coupling in methylene chloride. When hydroxybenztriazole esters are employed, both couplings are carried out in dimethyl formamide. Boc deprotection is accomplished with trifluoroacetic acid (TFA) in methylene chloride. Following completion of the synthesis, the peptides are deprotected and cleaved from the resin with anhydrous hydrogen fluoride containing 10% anisole. Cleavage of the side chain protecting group(s) and of the peptide from the resin is carried out at 0°C or below, preferably -20°C for thirty minutes followed by thirty minutes at 0°C. After removal of the HF, the peptide/resin is washed with ether, and the peptide extracted with neat TFA. TFA is removed by rotary evaporation and the resulting oil dissolved in glacial acetic acid and lyophilized. The crude cysteine containing peptide is then oxidized to the corresponding cysteic acid containing compound using performic acid at -10°C to 10°C, preferably at 0°C, as described by Stewart et al, Solid Phase Peptide Synthesis, pg. 113, Pierce Chemical Company, Rockford, Illinois, 1984. Purification is carried out by ion exchange chromatography on a Synchroprep S-300 (SynChrom Inc. Linden, Indiana) cation exchange column. The peptide is applied using a buffer of 20millimolar TRIS (pH 6.8) in 20% acetonitrile and eluted using a gradient of 0-0.3 molar sodium chloride in the same solvent. Compounds are further purified and desalted by reverse phase liquid chromatography on a Vydac C-18 (Separations Group, Hesperia, California) column using water:acetonitrile gradients, each phase containing 0.1% TFA. The desired fractions are pooled and lyophilized yielding the desired GRF PEPTIDE as its trifluoroacetate salt. The trifluoroacetate salt can be converted, if desired to other suitable salts, by well known ion exchange methods.

Example 1
Cya[25], Leu[27]bGRF(1-32)NH[2]; or Somatolibrin (human pancreatic islet) 25-(3-sulfo-L-alanyl)-27-L-leucyl-28-L-asparaginyl-32-glycinamide-33-de-L-glutamic acid-34-de-L-serine-35-de-L-asparagine-36-de-L-glutamine-37-de-L-glutamic acid-38-de-L-arginine-39-deglycine-40-de-L-alanine-41-de-L-arginine-39-deglycine-40-de-L-alanine-41-de-L-arganine-42-de-L-alanine-43-de-L-arganine-44-de-L-leucinamide; Cpd. No. 1

The synthesis of the GRF analog peptide having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Cya-Ile-Leu-Asn-Arg-Gln-Gln-Gly-NH[2] is conducted in a stepwise manner as in Procedure A.

The compound gave the expected amino acid analysis and the expected monoisotopic protonated molecular ion (3715.3) by fast atom bombardment mass spectroscopy.

Example 2
Cya[3,25], Leu[27]bGRF(1-32)NH[2]; or Somatolibrin (human pancreatic islet) 3-(3-sulfo-L-alanyl)-25-(3-sulfo-L-alanyl)-27-L-leucyl-28-L-asparaginyl-32-glycinamide-33-de-L-glutamic acid-34-de-L-serine-35-de-L-asparagine-36-de-L-glutamine-37-de-L-glutamic acid-38-de-L-arginine-39-deglycine-40-de-L-alanine-41-de-L-arginine-42-de-L-alanine-43-de-L-arginine-44-de-L-leucinamide; Cpd. No. 2

The synthesis of the GRF analog peptide having the formula:
H-Tyr-Ala-Cya-Ala-Ile-Phe-Thr-Asn-Ser-Tyr-Arg-Lys-Val-Leu-Gly-Gln-Leu-Ser-Ala-Arg-Lys-Leu-Leu-Gln-Cya-Ile-Leu-Asn-Arg-Gln-Gln-Gly-NH$_2$ is conducted in a stepwise manner as in Procedure A.

The compound gave the expected amino acid analysis and the expected monoisotopic protonated molecular ion (3751.1) by fast atom bombardment mass spectroscopy.

Example 3

Cya$^3$, Leu $^{27}$bGRF(1-32)NH$_2$; or Somatolibrin (human pancreatic islet) 3-(3-sulfo-L-alanyl)-27-L-leucyl-28-L-asparaginyl-32-glycinamide-33-de-L-glutamic acid-34-de-L-serine-de-L-asparagine 36-de-L-glutamine-37-de-L-glutamic acid-38-de-L-arganine-39-deglycine-40-de-L-alanine-41-de-L-arginine-42-de-L-alanine-43-de-L-arginine-44-de-L-leucinamide; Cpd. No. 3

The synthesis of the GRF analog peptide having the formula:
H-Tyr-Ala-Cya-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Leu-Asn-Arg-Gln-Gln-Gly-NH$_2$ is conducted in a stepwise manner as in Procedure A.

The compound gave the expected amino acid analysis and the expected monoisotopic protonated molecular ion (3715.4) by fast atom bombardment mass spectroscopy.

In addition to the foregoing specifically enumerated analogs, the following additional analogs have been synthesized in a stepwise manner as in Procedure A:

D-Ala$^2$, Cya$^3$, Leu$^{27}$-bGRF(1-32)NH$_2$ Cpd. #4

The compound gave the expected amino acid analysis.

Desamino-Tyr$^1$, Cya$^3$, Leu$^{27}$-bGRF(1-32)NH$_2$ Cpd. #5

The compound gave the expected amino acid analysis and the expected monoisotopic protonated molecular ion (3700.2) by fast atom bombardment mass spectroscopy.

Desamino-Tyr$^1$, D-Ala$^2$,, Cya$^3$, Leu$^{27}$-bGRF(1-32)NH$_2$ Cpd. #6

The compound gave the expected amino acid analysis and the expected monoisotopic protonated molecular ion (3700.1) by fast atom bombardment mass spectroscopy.

His$^1$, D-Ala$^2$, Cya$^3$, Leu$^{27}$-bGRF(1-32)NH$_2$ Cpd. #7

The compound gave the expected amino acid analysis.

D-Ala$^2$, Cya$^3$, Ala$^{15}$, Leu$^{27}$-bGRF(1-32)NH$_2$ Cpd. #8

The compound gave the expected amino acid analysis.

Desamino-Tyr$^1$, Cya$^3$, Ala$^{15}$, Leu$^{27}$-bGRF(1-32)NH$_2$ Cpd. #9

The compound gave the expected amino acid analysis and the expected monoisotopic protonated molecular ion (3714.2) by fast atom bombardment mass spectroscopy.

Desamino-Tyr$^1$, D-Ala$^2$, Cya$^3$, Ala$^{15}$, Leu$^{27}$-bGRF(1-32)NH$_2$ Cpd. #10

The compound gave the expected amino acid analysis and the expected monoisotopic protonated molecular ion (3714.2) by fast atom bombardment mass spectroscopy.

His$^1$, D-Ala$^2$, Cya$^3$, Ala$^{15}$, Leu$^{27}$-bGRF(1-32)NH$_2$ Cpd. #11

The compound gave the expected amino acid analysis.

DesaminoTyr$^1$, Cya$^3$, Ser$^8$, Arg$^{12}$, Ala$^{15}$, Leu$^{27}$, Ser$^{28}$, bGRF(1-32)NH$_2$ Cpd. #12

The compound gave the expected amino acid analysis and the expected monoisotopic protonated molecular ion (3725) by fast atom bombardment mass spectroscopy.

Desamino PHPG$^1$, Cya$^3$, Ser$^8$, Arg$^{12}$, Ala$^{15}$, Leu$^{27}$, Ser$^{28}$, bGRF(1-32)NH$_2$ Cpd. #13

The compound gave the expected amino acid analysis and the expected monoisotopic protonated molecular ion (3700) by fast atom bombardment mass spectroscopy.

DesaminoTyr$^1$, Ser$^8$, Arg$^{12}$, Ala$^{15}$, Cya$^{25}$, Leu$^{27}$, Ser$^{28}$, bGRF(1-32)NH$_2$ Cpd. #14

The compound gave the expected amino acid analysis and the expected monisotopic protonated molecular ion (3715) by fast atom bombardment mass spectroscopy.

Desamino PHPG$^1$, Ser$^8$, Arg$^{12}$, Ala$^{15}$, Cya$^{25}$, Leu$^{27}$, Ser$^{28}$, bGRF(1-32)NH$_2$ Cpd. #15

The compound gave the expected amino acid analysis and the expected monoisotopic protonated molecular ion (3700) by fast atom bombardment mass spectroscopy.

In addition to the foregoing analogs specifically enumerated analogs, the following analogs can be synthesized using the same solid-phase synthesis techniques set forth herein:
D-Ala$^2$, Arg$^{21}$, Cya$^{25}$, Ile$^{27}$ rGRF(1-43)OH Cpd. #16;
DesaminoTyr$^1$, Cya$^3$, Arg$^{12}$, Arg$^{21}$, Leu$^{27}$ porcine (GRF)1-44 OH Cpd. #17;
Cya$^{3,25}$ Leu$^{27}$ hGRF(1-44)OH Cpd. #18; and
DesaminoTyr$^1$, Cya$^3$, Arg$^{12}$, Ala$^{15}$, Arg$^{21}$, Leu$^{27}$, Arg$^{41}$ b(GRF)1-44OH Cpd. #19;
DesaminoTyr$^1$, Cya$^3$, Ser$^8$, Arg$^{12}$, Ala$^{15}$, Arg$^{21}$, Leu$^{27}$, Ser$^{28}$, Hse$^{33}$ bGRF(1-33)lactone Cpd. #20;
DesaminoTyr$^1$, Ser$^8$, Arg$^{12}$, Ala$^{15}$, Arg$^{21}$, Cya$^{25}$, Leu$^{27}$, Ser$^{28}$, Hse$^{33}$ bGRF(1-33)NH$_2$ Cpd #21;
Desamino PHPG$^1$, Cya$^3$, Ser$^8$, Arg$^{12}$, Ala$^{15}$, Arg$^{21}$, Leu$^{27}$, Ser$^{28}$, Hse$^{33}$ bGRF(2-33)NH(Ethyl) Cpd #22; and
Desamino PHPG$^1$, Ser$^8$, Arg$^{12}$, Ala$^{15}$, Arg$^{21}$, Cya$^{25}$, Leu$^{27}$, Ser$^{28}$, Hse$^{33}$ bGRF(2-33)OH Cpd #23.

In addition to preparation of GRF analogs by solid phase methods, certain analogs can be obtained by recombinant biosynthetic (DNA) methods or a combination of recombinant biosynthetic and synthetic methods, for example:
Cya$^{,3,25}$ Leu$^{27}$ hGRF(1-44)OH (Compound #18) can be prepared biosynthetically by the procedure

described for Leu[27] bGRF(1-44)OH (European Patent Application 0212531) with the following modifications:

The codons GAT (Asp[3]) and GAC (Asp[25]) are replaced by the codons TGT for Cys[3] and Cys[25] respectively. After expression of the protein and cleavage with cyanogen bromide in formic acid as described in the above European Patent Application, hydrogen peroxide is added to the solution at about $0°C$ to effect oxidation of the Cys residues to Cya. The peptide is then purified by the methods described.

DesaminoTyr[1], Cya[3], Arg[12], Ala[15], Arg[21], Leu[27], Arg[41] b(GRF)1-44OH (Compound 19) can be prepared by a combination of biosynthetic and synthetic steps as follows:

DNA coding for Met[1], Cys[3], Arg[12], Ala[15], Arg[21], Leu[27], Arg[41] b(GRF)1-44OH is prepared by well known methods in the art and inserted into an E. coli expression plasmid which is used to transform an E. coli host for expression of the desired polypeptide. The expressed polypeptides are isolated and cleaved by treatment with cyanogen bromide. After oxidation of Cys[3] to Cya[3] and purification, the Cya[3], Arg[12], Ala[15], Arg[21], Leu[27], Arg[41] b(GRF)2-44OH are dissolved in dimethylformamide and coupled with desaminotyrosine N-hydroxysuccinimide ester in the presence of a suitable tertiary amine base to give Compound #19.

Recombinant host microorganisms used in this invention are made by recombinant DNA techniques well known to those skilled in the art and set forth, for example, in Molecular Cloning, T. Maniatis, et al., Cold Spring Harbor Laboratory, (1982) and B. Perbal, A Practical Guide to Molecular Cloning, John Wiley & Sons (1984), which are incorporated herein by reference.

C-terminal Hse(lactone), HseOH and HseN($R_a$)($R_b$) analogs can be prepared by the methods disclosed in Kempe et al, BIO/TECHNOLOGY, Vol 4, pp 565-568 (1986).

In a bovine anterior pituitary cell culture assay, Compounds 1, 2 and 3 exhibited 0.53, 0.11 and 0.31 the potency of bGRF(1-44)NH₂. Leu[27] bGRF(1-32)NH₂ exhibited 0.87 the potency of bGRF(1-44)NH₂.

The synthetic peptides prepared in Examples 1, 2 and 3 (Compounds 1, 2 and 3 respectively), bGRF(1-44)NH₂ and Leu[27] bGRF(1-32)NH₂ were also given to meal-fed Holstein steers by intravenous injection (Procedure B) and the serum growth hormone (GH) response evaluated. No significant differences were observed between bGRF(1-44)NH₂ and Leu[27] bGRF(1-32)NH₂, between Leu[27] bGRF(1-32)NH₂ and Compounds 1, 2 and/or 3. In comparison to bGRF(1-44)NH₂ (100%), the area under the GH response curve was 122% for Leu[27] bGRF(1-32)NH₂, 100% for Compound #1, 106% for Compound #2, and 111% for Compound #3.

## PROCEDURE B

Holstein steers (265 +/- 5kg) were housed in individual stalls 18-20°C) and exposed to 16h light:8h darkness (lights on at 0400h). Steers are meal-fed once daily at 1000h and trained to consume their total daily feed intake in 2 h. The diet consisted of rolled corn plus a mineral/vitamin premix and alfalfa hay cubes fed at 160% maintenance energy requirements on a bodyweight basis. Steers gained approximately 0.75 kg/h/d.

In a 5 x % Latin Square design 10 steers were assigned randomly to receive the following treatments. (a) bGRF(1-44)NH₂, (b) Leu[27] bGRF(1-32)NH₂, (c) Compound #3, (d) Compound #1, and (e) Compound #2. The GRFs were dissolves in saline (10µg/ml) and administered at 3nM/100 kg bodyweight as single iv injection at 0800 h, 2 h between feeding.

A cannula was inserted into a jugular vein at least one day before treatment. Blood (6 ml) was collected at 20-min interval from 40 min before until 120 min after injection with additional samples collected at 5, 10 and 15 min after injection. Serum obtained from the samples was assayed from GH concentration.

The number of steers responding to injection fo the various GRFs tested (P=0.42) ranged from 9 of 10 to 10 of 10 depending on the treatment.

In a subsequent bovine anterior pituitary cell culture assay, Compounds 3, 4, 5, 6, 7, 8, 9, 10 and 11 exhibited 0.41, 0.29, 1.01, 0.26, 0.06, 1.34, 2.24, 1.40 and 0.28 the potency of bGRF(1-44)NH₂. Leu[27] bGRF(1-32)NH₂ exhibited 0.35 the potency of bGRF(1-44)NH₂.

In another bovine anterior pituitary cell culture assay, Cpds 12, 13, 14 and 15 exhibited 0.54, 1.98, 1.21, and 0.97 the potency of bGRF(1-44)NH₂. Leu[27] bGRF(1-32)NH₂ exhibited 0.85 the potency of bGRF(1-44)NH₂.

In addition to the foregoing bovine pituitary cell culture assays, a number of compounds of this invention have been assayed for proteolytic stability in bovine plasma and have been found to be more stable than Leu[27] bGRF(1-32)NH2. Various compounds were assayed by HPLC using a modification of the method described by Frohman (J. Clin. Invest. 78,906-913 (1986). Upon incubation in bovine plasma for 1 hour, only 47% of Leu[27] bGRF(1-32)NH₂ remained. Under the same incubation conditions with compound 3, 42% remained; compound 4, 76% remained; compound 5, 76% remained; compound 6, 51% remained; compound 7, 69% remained; compound 8, 95% remained; compound 9, 81% remained; compound 11, 86% remained; compound 12, 67% remained; compound 13, 63% remained; compound 14, 65% remained; compound 15, 65% remained.

Using procedure B, compounds were tested for growth hormone release in steers. In comparison to the standard bGRF(1-44)NH₂ (Area 100%), the test compounds gave the following relative growth hormone areas:
Compound 4, 233%; compound 5, 140%; compound 6, 160%; compound 7, 126%; compound 8, 307%; compound 9, 260%; and compound 11, 173%.

Based on the bovine pituitary cell culture assays described herein and a compounds proteolytic stability in bovine plasma, compounds with high potency and good stability such as Compound 8 and 9 are preferred. It is contemplated that Compound 20, which can be prepared by a combination of biosynthetic and synthetic steps, will also exhibit high potency and good stability and is therefor preferred.

The most preferred compounds are Cpd. #'s 21, 22, and/or 23.

All of the synthetic GRF peptides of the subject invention, including the peptides prepared in the Examples, are considered to be biologically active and useful for stimulating the release of GH by the pituitary.

Dosages between about 50 nanograms and about 5 micrograms of these peptides per Kg. of body weight are considered to be particulary effective in causing GH secretion.

Stimulation of GH secretion by such peptides should result in an attendant increase in growth for humans, bovine and other animals with normal GH levels. Moreover, administration should alter body fat content and modify other GH-dependent metabolic, immunologic and developmental processes. For example, these analogs may be useful as a means of stimulating anabolic processes in human beings under circumstances such as following the incurring of burns. As another example, these analogs may be administered to commercial warm-blooded animals such as chickens, turkeys, pigs, goats, cattle and sheep, and may be used in aquaculture for raising fish and other cold-blooded marine animals, e.g., sea turtles and eels, and amphibians, to accelerate growth and increase the ratio of protein to fat gained by feeding effective amounts of the peptides.

Daily dosages of between 50 nanograms/Kg and about 50 micrograms/Kg body weight are considered to be particularly effective in increasing lactation and growth.

For administration to humans and animals, these synthetic peptides should have a purity of at least about 93% and preferably at least 98%.

These synthetic peptides or the nontoxic salts thereof, combined with a pharmaceutically or veterinarily acceptable carrier to form a pharmaceutical composition, preferably as sustained release formulations, may be administered to animals, including humans, either intravenously, subcutaneously, intramuscularly, percutaneously, e.g. intranasally. The administration may be employed by a physician to stimulate the release of GH where the host being treated requires such therapeutic treatment. The required dosage will vary with the particular condition being treated, with the severity of the condition and with the duration of desired treatment.

Such peptides are often administered in the form of nontoxic salts, such as acid addition salts or metal complexes, e.g., with zinc, iron or the like (which are considered as salts for purposes of this application). Illustrative of such acid addition salts are hydrochloride, hydrobromide, sulphate, phosphate, maleate, acetate, citrate, benzoate, succinate, malate. ascorbate, tartrate and the like. If the active ingredient is to administered by intravenous administration in isotonic saline, phosphate buffer solutions or the like may be effected.

The peptides should be administered to humans under the guidance of a physician, and pharmaceutical compositions will usually contain the peptide in conjunction with a conventional, solid or liquid, pharmaceutically-acceptable carrier. Usually, the parental dosage will be from about 100 nanograms to about 50 micrograms of the peptide per kilogram of the body weight of the host.

Although the invention has been described with regard to its preferred embodiments, it should be understood that various changes and modifications as would be obvious to one having the ordinary skill in this art may be made without departing from the scope of the invention which is set forth in the claims appended hereto. For example, modifications in the peptide chains, particularly deletions of one or two residues beginning at the C-terminus of the peptide, can be made in accordance with known experimental practices to date to create peptides that retain very substantial portions of the biological potency of the peptide, and such peptides are considered as being within the scope of the invention. Moreover, additions may be made to the C-terminus, and/or generally equivalent residues can be substituted for naturally occurring residues, as is known in the overall art of peptide chemistry, to produce other analogs, having increased resistance to proteolysis, for example, and also having at least a substantial portion of the potency of the claimed polypeptide, without deviating from the scope of the invention, such as those illustrated by Compounds 1-15. Likewise known substitutions in the carboxyl moiety at the C-terminus, e.g. a lower alkyl amide, also produce equivalent molecules.

**Claims**

1. A GRF PEPTIDE wherein a cysteic acid residue (Cya) is substituted for the amino acid in position $R_3$ and/or $R_{25}$.

2. A GRF PEPTIDE according to claim 1 wherein a cysteic acid residue (Cya) is substituted for Asp or Glu in position $R_3$ and/or $R_{25}$.

3. A GRF PEPTIDE according to claim 2 having the formula:
H-$R_1$-$R_2$-$R_3$-Ala-$R_5$-$R_6$-$R_7$-$R_8$-$R_9$-$R_{10}$-$R_{11}$-$R_{12}$-$R_{13}$-$R_{14}$-$R_{15}$-Gln-$R_{17}$-$R_{18}$-$R_{19}$-$R_{20}$-$R_{21}$-$R_{22}$-$R_{23}$-$R_{24}$-$R_{25}$-$R_{26}$-$R_{27}$-$R_{28}$-$R_{29}$-Gln-Gln-Gly-Glu-Ser-Asn-Gln-Glu-Arg-Gly-Ala-$R_{40}$-Y
wherein $R_1$ is Tyr, D-Tyr, His, D-His, Phe, D-Phe, (optionally substituted with either a $C^\alpha$Me or $N^\alpha$Me substituent), DesaminoTyr, Desamino PHPG, or DesaminoHis; $R_2$ is Ala or D-Ala optionally substituted with either a $C^\alpha$Me or $N^\alpha$Me substituent, or Ser; $R_3$ is Asp, D-Asp, Glu or D-Glu; $R_5$ is Ile or Leu; $R_6$ is Phe or Tyr; $R_7$ is Ser or Thr; $R_8$ is Asn, D-Asn, Ser, D-Ser or Thr; R9 is Ala or Ser; $R_{10}$ is Tyr, D-Tyr or Phe; $R_{11}$ is Arg or Lys; $R_{12}$ is Arg or Lys; $R_{13}$ is Ile, Leu or Val; $R_{14}$ is Leu, D-Leu, Ile or Val; $R_{15}$ is Ala, D-Ala, Gly, Gln or Leu; $R_{17}$ is Ala, Leu or D-Leu; $R_{18}$ is Ala, Leu, Ser or Tyr; $R_{19}$ is Ala or Ser; $R_{20}$ is Arg, or Lys; $R_{21}$ is Arg, or Lys; $R_{22}$ is Ala, Leu

or Ile; $R_{23}$ is Leu, D-Leu, Ile or Val; $R_{24}$ is Gln or His; $R_{25}$ is Asp or Glu; $R_{26}$ is Ile, Leu or Val; $R_{27}$ is Ala, D-Ala, Nle, Val, Leu or Ile; $R_{28}$ is Asn, Ser or Ala; $R_{29}$ is Arg, D-Arg, Lys; $R_{40}$ is Arg, Arg-Ala, Arg-Ala-Arg or Arg-Ala-Arg-Leu; and Y signifies the carboxyl moiety of the amino acid residue at the C-terminal and is the radical -COOR$_a$, -CR$_a$O, -CONHNHR$_a$, -CON(R$_a$)(R$_b$) or -CH$_2$OR$_a$, with R$_a$ and R$_b$ being lower alkyl or hydrogen; wherein cysteic acid residue (Cya) is substituted for Asp or Glu in position $R_3$ and/or $R_{25}$; or a biologically active fragment thereof extending from R at the N-terminus to a residue in any of positions 27 through 39 as its C-terminus; a non-toxic salt of the foregoing; or a Hse(lactone), HseOH or HseN(R$_a$)(R$_b$) of the foregoing.

4. A GRF PEPTIDE having the formula of Claim 3 wherein $\acute{R}_3$ is a cysteic acid residue (Cya).

5. A GRF PEPTIDE having the formula of Claim 3 wherein $R_{25}$ is a cysteic acid residue (Cya).

6. A GRF PEPTIDE having the formula of Claim 3 wherein $R_3$ and $R_{25}$ are cysteic acid residues (Cya).

7. The GRF PEPTIDE of Claim 1 having the formula:
H-Tyr-Ala-Cya-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Leu-Asn-Arg-Gln-Gln-Gly-NH$_2$.

8. The GRF PEPTIDE of Claim 1 having the formula:
H-Tyr-Ala-Cya-Ala-Ile-Phe-Thr-Asn-Ser-Tyr-Arg-Lys-Val-Leu-Gly-Gln-Leu-Ser-Ala-Arg-Lys-Leu-Leu-Gln-Cya-Ile-Leu-Asn-Arg-Gln-Gln-Gly-NH$_2$.

9. THE GRF PEPTIDE of Claim 1 having the formula:
H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Cya-Ile-Leu-Asn-Arg-Gln-Gln-Gly-NH$_2$.

10. THE GRF PEPTIDE of Claim 1 having the formula:
DesaminoTyr-Ala-Cya-Ala-Ile-Phe-Thr-Ser-Ser-Tyr-Arg-Arg-Val-Leu-Ala-Gln-Leu-Ser-Ala-Arg-Arg-Leu-Leu-Gln-Asp-Ile-Leu-Ser-Arg-Gln-Gln-Gly-Hse-lactone.

11. The GRF PEPTIDE of Claim 1 having the formula:
DesaminoTyr-Ala-Asp-Ala-Ile-Phe-Thr-Ser-Ser-Tyr-Arg-Arg-Val-Leu-Ala-Gln-Leu-Ser-Ala-Arg-Arg-Leu-Leu-Gln-Cya-Ile-Leu-Ser-Arg-Gln-Gln-Gly-Hse-NH$_2$.

12. The GRF PEPTIDE of Claim 1 having the formula:
N-[(para-hydroxyphenyl)acetyl]-Ala-Cya-Ala-Ile-Phe-Thr-Ser-Ser-Tyr-Arg-Arg-Val-Leu-Ala-Gln-Leu-Ser-Ala-Arg-Arg-Leu-Leu-Gln-Asp-Ile-Leu-Ser-Arg-Hse-NH(Ethyl).

13. The GRF PEPTIDE of Claim 1 having the formula:
N-[(para-hydroxyphenyl)acetyl]-Ala-Asp-Ala-Ile-Phe-Thr-Ser-Ser-Tyr-Arg-Arg-Val-Leu-Ala-Gln-Leu-Ser-Ala-Arg-Arg-Leu-Leu-Gln-Cya-Ile-Leu-Ser-Arg-Gln-Gln-Gly-Hse-OH.

14. A method of stimulating the release of growth hormone in an animal, which comprises administering to said animal an effective amount of a peptide of the formula:
H-$R_1$-$R_2$-$R_3$-Ala-$R_5$-$R_6$-$R_7$-$R_8$-$R_9$-$R_{10}$-$R_{11}$-$R_{12}$-$R_{13}$-$R_{14}$-$R_{15}$-Gln-$R_{17}$-$R_{18}$-$R_{19}$-$R_{20}$-$R_{21}$-$R_{22}$-$R_{23}$-$R_{24}$-$R_{25}$-$R_{26}$-$R_{27}$-$R_{28}$-$R_{29}$-Gln-Gln-Gly-Glu-Ser-Asn-Gln-Glu-Arg-Gly-Ala-$R_{40}$-Y
wherein $R_1$ is Tyr, D-Tyr, His, D-His, Phe, D-Phe, (optionally substituted with either a C$^\alpha$Me or N$^\alpha$Me substituent), DesaminoTyr, Desamino PHPG, or DesaminoHis; $R_2$ is Ala or D-Ala optionally substituted with either a C$^\alpha$Me or N$^\alpha$Me substituent, or Ser; $R_3$ is Asp, D-Asp, Glu or D-Glu; $R_5$ is Ile or Leu; $R_6$ is Phe or Tyr; $R_7$ is Ser or Thr; $R_8$ is Asn, D-Asn, Ser, D-Ser or Thr; R9 is Ala or Ser; $R_{10}$ is Tyr, D-Tyr or Phe; $R_{11}$ is Arg or Lys; $R_{12}$ is Arg or Lys; $R_{13}$ is Ile, Leu or Val; $R_{14}$ is Leu, D-Leu, Ile or Val; $R_{15}$ is Ala, D-Ala, Gly, Gln or Leu; $R_{17}$ is Ala, Leu or D-Leu; $R_{18}$ is Ala, Leu, Ser or Tyr; $R_{19}$ is Ala or Ser; $R_{20}$ is Arg, or Lys; $R_{21}$ is Arg, of Lys; $R_{22}$ is Ala, Leu or Ile; $R_{23}$ is Leu, D-Leu, Ile or Val; $R_{24}$ is Gln or His; $R_{25}$ is Asp or Glu; $R_{26}$ is Ile, Leu or Val; $R_{27}$ is Ala, D-Ala, Nle, Val, Leu or Ile; $R_{28}$ is Asn, Ser or Ala; $R_{29}$ is Arg, D-Arg, Lys; $R_{40}$ is Arg, Arg-Ala, Arg-Ala-Arg or Arg-Ala-Arg-Leu; and Y signifies the carboxyl moiety of the amino acid residue at the C-terminal and is the radical -COOR$_a$, -CR$_a$O,-CONHNHR$_a$, -CON(R$_a$)(R$_b$) or -CH$_2$OR$_a$, with R$_a$ and R$_b$ being lower alkyl or hydrogen; wherein cysteic acid residue (Cya) is substituted for Asp or Glu in position $R_3$ and/or $R_{25}$; or a biologically active fragment thereof extending from R at the N-terminus to a residue in any of positions 27 through 39 as its C-terminus; a non-toxic salt of the foregoing; or a Hse(lactone), HseOH or HseN(R$_a$)(R$_b$) of the foregoing.

15. A composition for stimulating the release of growth hormone in an animal comprising an effective amount of a peptide of the formula:
H-$R_1$-$R_2$-$R_3$-Ala-$R_5$-$R_6$-$R_7$-$R_8$-$R_9$-$R_{10}$-$R_{11}$-$R_{12}$-$R_{13}$-$R_{14}$-$R_{15}$-Gln-$R_{17}$-$R_{18}$-$R_{19}$-$R_{20}$-$R_{21}$-$R_{22}$-$R_{23}$-$R_{24}$-$R_{25}$-$R_{26}$-$R_{27}$-$R_{28}$-$R_{29}$-Gln-Gln-Gly-Glu-Ser-Asn-Gln-Glu-Arg-Gly-Ala-$R_{40}$-Y
wherein $R_1$ is Tyr, D-Tyr, His, D-His, Phe, D-Phe, (optionally substituted with either a C$^\alpha$Me or N$^\alpha$Me substituent), DesaminoTyr, Desamino PHPG, or DesaminoHis; $R_2$ is Ala or D-Ala optionally substituted with either a C$^\alpha$Me or N$^\alpha$Me substituent, or Ser; $R_3$ is Asp, D-Asp, Glu or D-Glu; $R_5$ is Ile or Leu; $R_6$ is Phe or Tyr; $R_7$ is Ser or Thr; $R_8$ is Asn, D-Asn, Ser, D-Ser or Thr; R9 is Ala or Ser; $R_{10}$ is Tyr, D-Tyr or Phe; $R_{11}$ is Arg or Lys; $R_{12}$ is Arg or Lys; $R_{13}$ is Ile, Leu or Val; $R_{14}$ is Leu, D-Leu, Ile or Val; $R_{15}$ is Ala, D-Ala, Gly, Gln or Leu; $R_{17}$ is Ala, Leu or D-Leu; $R_{18}$ is Ala, Leu, Ser or Tyr; $R_{19}$ is Ala or Ser; $R_{20}$ is Arg, or Lys; $R_{21}$ is Arg, or Lys; $R_{22}$ is Ala, Leu or Ile; $R_{23}$ is Leu, D-Leu, Ile or Val; $R_{24}$ is Gln or His; $R_{25}$ is Asp or Glu; $R_{26}$ is Ile, Leu or Val; $R_{27}$

is Ala, D-Ala, Nle, Val, Leu or Ile; $R_{28}$ is Asn, Ser or Ala; $R_{29}$ is Arg, D-Arg, Lys; $R_{40}$ is Arg, Arg-Ala, Arg-Ala-Arg or Arg-Ala-Arg-Leu; and Y signifies the carboxyl moiety of the amino acid residue at the C-terminal and is the radical -COOR$_a$, -CR$_a$O, -CONHNHR$_a$, -CON(R$_a$)(R$_b$) or -CH$_2$OR$_a$, with R$_a$ and R$_b$ being lower alkyl or hydrogen; wherein cysteic acid residue (Cya) is substituted for Asp or Glu in position $R_3$ and/or $R_{25}$; or a biologically active fragment thereof extending from R at the N-terminus to a residue in any of positions 27 through 39 as its C-terminus; a non-toxic salt of the foregoing; or a Hse(lactone), HseOH or HseN(R$_a$)(R$_b$) of the foregoing.

European Patent
Office

EUROPEAN SEARCH REPORT

Application Number

EP 89 30 0827

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 177 819 (F. HOFFMANN) <br> * Whole document * <br> --- | 1 | C 07 K 7/10 <br> A 61 K 37/02 |
| A | J. MED. CHEM., vol. 28, no.2, 1985, pages 181-185, American Chemical Society; D.H. COY et al.: "Structure-activity studies on the N-terminal region of growth hormone releasing factor" <br> ----- | 1 | |

TECHNICAL FIELDS SEARCHED (Int. Cl.4)

C 07 K
A 61 K

The present search report has been drawn up for all claims

| Place of search | Date of completion of the search | Examiner |
|---|---|---|
| THE HAGUE | 28-03-1989 | RAJIC M. |

EPO FORM 1503 03.82 (P0401)